# EUROPEAN PATENT APPLICATION

(11) **EP 1 736 548 A1**
(43) Date of publication of application: **27.12.2006**
(21) Application number: 06012685.1
(22) Date of filing: 20.06.2006
(51) Int. Cl.: C12P 7/06, C12P 7/48, C12P 7/16, C12P 7/56, C12P 3/00, C12P 19/02

(54) **Use of corn with low gelatinization temperature for production of fermentation-based products**

(30) Priority: 22.06.2005 US 692999 P; 05.04.2006 US 398409
(71) Applicant: National Starch and Chemical Investment Holding Corporation, New Castle, Delaware 19720 (US)
(72) Inventor: Ostrander, Brad, Indianapolis Indiana 46205 (US)
(74) Representative: Held, Stephan

(57) **Abstract**

The present invention provides a method of producing fermentation-based products, such as ethanol and citric acid, from corn in which the starch has a low gelatinization temperature, particularly a waxy maize plant which is homozygous or heterozygous for the recessive sugary-2 allele.

## Description

The application claims priority to US Provisional Application No. 60/692,999.

### BACKGROUND OF THE INVENTION

The present invention relates to an improved method of producing fermentation-based products derived from corn containing starch with a lower gelatinization temperature.

Corn or maize is grown for many reasons including its use in food and industrial applications. Ethanol (ethyl alcohol, grain alcohol, EtOH) is a clear, colorless liquid with a characteristic, agreeable odor that is desirable for use in motor vehicle fuels to control pollution. Traditionally, corn has been used, along with other crops such as sugar beets and sugar cane, to produce ethanol.

Corn has traditionally been processed by one of two methods. The wet milling process involves soaking or steeping the corn to recover the oil prior to processing, leaving behind a corn meal. In the dry milling process, the whole kernel may be ground and then water is added to form a mash. In either case, where the corn is to be used to produce ethanol, the meal or mash is treated with an enzyme such to convert the starch contained in the corn to sugars. The enzyme treated mash or meal is then fermented using yeast. The yeast converts the sugar to ethanol and carbon dioxide. Once the ethanol and carbon dioxide have been separated, such as by distillation, the remaining non-fermentable part of the corn can be processed to recover other nutrients and can also be processed into animal feed.

Industry advocates are continually in search of better corn-based feedstocks and methods to produce high grade ethanols efficiently. Surprisingly, it has now been discovered that corn in which the starch has a low gelatinization temperature may be used as a feedstock to produce fermentation-based products more efficiently with lower energy input.

### SUMMARY OF THE INVENTION

The present invention provides a method of producing fermentation-based products, such as ethanol, citric acid, butanol, and hydrogen from corn in which the starch has a low gelatinization temperature. The method comprises mixing the corn with a micro-organism capable of fermenting a carbon source to produce fermentation-based products.

Gelatinization temperature, as used herein, is intended to mean the initial gelatinization temperature (To) as measured using differential scanning colorimetry by the method disclosed in Example1 of the Examples section.

Unless otherwise defined, all technical and scientific terms and abbreviations used herein have the same meaning as commonly understood by one of ordinary skill in the art to which this invention pertains. Although methods and materials similar or equivalent to those described herein can be used in the practice of the present invention, suitable methods and materials are described below without intending that any such methods and materials limit the invention described herein. Additional features and advantages of the invention will be apparent from the following description of illustrative embodiments of the invention and from the claims.

### DETAILED DESCRIPTION OF THE INVENTION

Corn seed or grain (hereinafter "grain") harvested from any of several different types of corn plants is useful in the invention. These types of corn plants are, for example, hybrids, inbreds, transgenic plants, genetically modified plants or a specific population of plants. In one embodiment, the corn is derived from a waxy maize plant which endosperm tissue is heterozygous (either one or two doses) for the recessive sugary-2 allele. In another embodiment, the corn is derived from a waxy maize plant which endosperm tissue is homozygous for the recessive sugary-2 allele. Other embodiments include those in which the corn is derived from a plant which endosperm tissue is homozygous for the recessive sugary-2 allele, dull-sugary2, dull-sugary2-waxy, amylose extender-dull-waxy, amylose extender-sugary2, and dosage combinations thereof. All these genotypes are known in the art and corn or seed for producing corn plants with these genotypes are available commercially.

The waxy genotype (designated as wx) is well known in the art and the waxy gene is located at position 59 of Chromosome 9 of corn (See M. G. Nueffer, L. Jones, and M. Zuber, "The Mutants of Maize" (Crop Science Society of America, Madison, WI, 1968), pp. 72 and 73.). The waxy genotype imparts to the corn plant the ability to produce a starch which consists primarily or totally of amylopectin, and the phenotype, or physical expression, of the endosperm of the waxy genotype is opaque with a hard waxy texture. In particular, waxy genotypes are those in which there has been a mutation of the granular bound starch synthase (GBSS). Common waxy maize is grown for many purposes and is commercially available.

The sugary-2 genotype (designated as su₂) is known to alter the carbohydrate composition of the maize endosperm, and the sugary-2 gene is located at position (57) of Chromosome 6 (Ibid). The double-recessive mutant of the waxy sugary-2 genotype is also known. Waxy maize which endosperm is homozygous for the sugary-2 allele is described in the literature, including U.S. Pat. Nos. 4,428,972 and 4,615,888 and is commercially available from National Starch and Chemical Company.

The genotype of the plant may also be obtained by translocation, inversion, transformation or any other method of gene or chromosome engineering to include variations thereof whereby the properties of the starch of this invention are obtained. In addition, starch extracted from a plant grown from artificial mutations and variations of the above generic composition which may be produced by known standard methods of mutation breeding is also applicable herein.

The genotype of the plant from which the starch is extracted may be obtained by standard breeding techniques. To obtain the double-recessive mutant of the wxsu2 genotype in maize in a usual manner, one may, for example, cross a waxy mutant (wx) with a sugary-2 mutant (su2), and thereafter self pollinate the first generation single cross (Wx wx Su2 su2) to theoretically recover the double mutant in a 15:1 ratio from a segregating ear. The starch utilized in this invention may be obtained from inbred lines, but it is more desirable that the starch be obtained from hybrids derived from inbreds containing the wxsu2 double-recessive mutant, ordinarily because of higher yields and other factors. Field production of maize plants with endosperms that have one dose of the recessive sugary-2 allele may be carried out by crossing female waxy maize plants with the dominant Sugary-2 allele with male waxy maize plants with the recessive sugary-2 allele in homozygous condition. A typical planting arrangement is one male row to seven female rows. The female rows are either detasseled or rendered male sterile through various other means known in the art such as cytoplasmic or genetic means. Field production of maize plants with endosperms that have two doses of the recessive sugary-2 allele may carried out by crossing female waxy maize plants that are homozygous recessive for the sugary-2 allele with male waxy maize plants with the dominant Sugary-2 allele. Planting arrangement and rendering the female plants male sterile would be similar to one dose production.

The corn seed used in this invention must contain starch with a low gelatinization temperature. In one embodiment, the gelatinization temperature is less than about 60 °C, in a second embodiment less than about 55 °C and in a third embodiment less than about 50 °C.

The process for producing ethanol and other fermentation-based products are well known in the art. Ethanol is typically produced from corn meal or mash that contain fermentable sugars or constituents which can be converted into sugars. In one conventional process, the meal or mash is saccarified in the presence of a glucoamylase to obtain hydrolyzed starch and sugars, and fermented by yeast to obtain ethanol. The ethanol is then recovered. The corn meal or mash may be liquefied in the presence of an alpha-amylase prior to saccharification. A protease may be introduced to the liquefied mash during saccharification and/or to the hydrolyzed starch and sugars during the fermentation mash to allow ethanol fermentation by yeast in the presence of higher dry solids mash levels.

The corn may be used in its whole kernel state; that is a kernel that has not been separated into its constituent parts, e.g. the hull, endosperm, tipcap, pericarp, and germ or in its separate states as known in the art. However, it is necessary that enough starch remains to support the fermentation. The whole corn may be ground, crushed, cracked, flaked, or abraded, for example by using a hammer mill or a roller mill. In one embodiment, solvent extracted corn meal is used for fermentation-based production. In another embodiment, corn mash is used for fermentation-based production. Blends of different corns and/or different constituent parts may also be used to produce fermentation-based products.

Liquefaction is conventionally achieved by adding water to the corn, and adjusting the pH to about 6 using base, such as lime. Alpha-amylase is then added to liquefy the starch. Nitrogen yeast nutrient may also be added. Steam injection may be used to heat the corn to a temperature of about 88-120 °C to gelatinize and pasteurize the starch. The corn is then held at a temperature efficient for the amylase used to convert the starch to complex sugars, conventionally 63 to 95 °C for about 2-4 hours. The corn component is conventionally about 30% by weight, but higher solids may be used.

The corn is then conventionally cooled prior to saccharification to a temperature efficient for enzyme activity of the glucoamylase, for example to about 60 °C. The pH is adjusted to a pH efficient for the enzyme activity, for example to about 4.4, such as by sulfuric acid, and the glucoamylase is added. Pullulanase may optionally be added to hydrolyze the 1,6-bonds. Saccarification conventionally is achieved within six hours. Optionally, liquefication and saccharification may be achieved simultaneously.

The corn is then conventionally cooled further to allow efficient yeast fermentation, for example to 32 °C. Fermentation typically takes about 46-50 hours, with temperature and pH being maintained.

The fermentation-based products made using this invention are any of those known in the art, including without limitation ethanol, citric acid, butanol, hydrogen, lactic acid, vitamins, and soluble sugars. In one embodiment, the fermentation-based product produced in ethanol and in another is citric acid.

Generally, after fermentation, the ethanol is recovered by distillation and dehydration to recover ethanol that is over 99.9% pure. A small amount of gasoline may be added to denature the alcohol.

Nutrients used in the cultivation of these and other microorganisms include, for example, backset, yeast extract, corn steep liquor, starch, glucose, alcohols, ketones, and as a nitrogen source, peptone, soybean powder, ammonium chloride, ammonium sulfate, ammonium nitrate, extracted corn meal, or urea. Various salts, such as NaCl and ammonium sulfate, and trace elements may also be included in media for the culture of microorganisms. It may also be advantageous to add some other enzymes to the liquefied mash during saccharification and/or to add the enzymes to the hydrolyzed starch and sugars during fermentation. Examples of such enzymes are cellulases, hemicellulase, phosphatase, exo- and endoglucanases, and xylanase.

The following embodiments are presented to further illustrate and explain the present invention and should not be taken as limiting in any regard.
1. In a method of producing fermentation-based products from corn of the type wherein the corn is mixed with a micro-organism capable of fermenting a carbon source to produce fermentation-based products, the improvement comprising using a corn comprising a starch characterized by a gelatinization temperature of less than about 60 °C.
2. The method of embodiment 1, wherein the corn is from a maize plant which endosperm is homozygous recessive for the allele selected from the group consisting of sugary2, sugary2-waxy, dull-sugary2, dull-sugary2-waxy, amylose extender-dull-waxy, amylose extender-sugary2, and dosage combinations thereof
3. The method of embodiment 2, wherein the corn is from a waxy maize plant which endosperm is homozygous for the recessive sugary-2 allele.
4. The method of embodiment 1, wherein the corn is from a waxy maize plant which endosperm is heterozygous for the recessive sugary-2 allele, one dose.
5. The method of embodiment 1, wherein the corn is from a waxy maize plant which endosperm is heterozygous for the recessive sugary-2 allele, two doses.
6. The method of embodiment 1, wherein the starch is characterized by a gelatinization temperature of less than about 55 °C.
7. The method of embodiment 1, wherein the starch is characterized by a gelatinization temperature of less than about 50 °C.
8. The method of embodiment 1, wherein the fermentation-based product is selected from the group consisting of ethanol, citric acid, butanol, hydrogen, lactic acid, vitamins, and soluble sugars.
9. The method of embodiment 8, wherein the fermentation-based product is ethanol.
10. The method of embodiment 8, wherein the fermentation based product is citric acid.

### EXAMPLES

The following examples are presented to further illustrate and explain the present invention and should not be taken as limiting in any regard. All percents used are on a weight/weight basis.

### Example 1 - Gelatinization of a variety of corn starches

Differential scanning calorimetry was used to investigate the characteristics of gelatinization of waxy starches with zero to three doses of the recessive sugary-2 gene. 10 mg starch were weighed into stainless steel pans. Water was added at a ratio of one part starch to two parts water. The samples were then heated from 30° C. to 102°C, at a heating rate of 10° C./min. The results are shown in the Table 1 below.

**TABLE 1**

| **Gelatinization Data** | | | | |
|---|---|---|---|---|
| **dosing** | **Onset T (°C)** | **Peak T (°C)** | **Offset T (°C)** | **Delta H (J/g)** |
| **0** | **65.5** | **73.3** | **88.0** | **16.72** |
| **1** | **60.9** | **68.8** | **84.5** | **14.98** |
| **2** | **57.8** | **66.4** | **84.5** | **14.11** |
| **3** | **49.1** | **56.6** | **77.3** | **11.31** |

### Example 2 ― DSC characterization of a variety of starches

Inouchi, et al (Starke 43(12) S468-472 (1991) characterized a variety of starches in their article "DSC Characteristics of Gelatinization of Starches of Single-, Double, and Triple- Mutants and Their Normal Counterpart in the Inbred Oh43 Maize (*Zea Mays. L*) Background." Starch was weighed into an aluminum pan and distilled water was added at a ratio of one part starch to two parts water. The samples were then heated at a heating rate of 2° C./min. The results are shown in the Table 2 below.

**TABLE 2**

| **Gelatinization Data** | | | | |
|---|---|---|---|---|
| **Genotype** | **Onset T (°C)** | **Peak T (°C)** | **Offset T (°C)** | **Delta H (cal/g)** |
| **Dent** | **61** | **66** | **72** | **3.6** |
| **wx** | **62** | **69** | **81** | **4.6** |
| **ae** | **65** | **83** | **94** | **--** |
| **du** | **64** | **68** | **75** | **3.4** |
| **su₂** | **45** | **53** | **62** | **1.2** |
| **du wx** | **63** | **72** | **82** | **4.0** |
| **su₂ wx** | **43** | **49** | **59** | **1.7** |
| **ae wx o** | **69** | **78** | **92** | **4.0** |
| **du su₂** | **47** | **53** | **64** | **0.9** |
| **du su₂ wx** | **42** | **48** | **55** | **1.8** |
| **ae du** | **60** | **68** | **77** | **2.4** |
| **ae du wx** | **50** | **70** | **76** | **3.4** |
| **ae su₂** | **51** | | **87** | **1.7** |

### Example 3 - Ethanol production using meal

a. Corn meal produced from corn derived from a waxy maize plant which is homozygous for the recessive sugary-2 allele (45 grams) is added to a 125 ml flask. Yeast extract is added at 1 g/L to ensure that nitrogen was not limiting. Cultures are inoculated with 10% inoculum from overnight yeast cultures (a typical Altech ethanol yeast of Saccharomyces cerevisiae) and incubations proceeded for 42 h at 30 °C on a rotary shaker at 125 rpm. Dextrose consumption and ethanol production are monitored by HPLC.
b. Example 3a is repeated using corn meal produced from corn derived from a waxy maize plant which is heterozygous for the recessive sugary-2 allele (one dose).
c. Example 3a is repeated using corn meal produced from corn derived from a waxy maize plant which is heterozygous for the recessive sugary-2 allele (two doses).
Ethanol production is similar to that by yeast grown on waxy and dent corn grain samples with less energy input. Example 2a required the lowest energy input.

### Example 4 ― Ethanol production using mash

a. Ground whole corn derived from a waxy maize plant which is homozygous for the recessive sugary-2 allele is obtained commercially. For liquefaction, 1740 gm of ground corn is added to 4500 ml of tap water. To this slurry is added 0.99 gm of CaCl₂·2H₂O. The slurry is then placed in 68 °C, and the pH adjusted to 6.2-6.4. Then, while constantly stirring, 0.6 ml of the enzyme Taka-Therm.RTM. Il is added to the slurry and then incubated at 68 °C for one hour. The enzyme Taka-Therm.RTM. Il is a liquid thermal stable bacterial (Bacillus licheniformis var.) alpha-amylase commercially available from Solvay Enzymes, Inc. No noticeable gelatinization is observed during the incubation. The slurry is then placed on a hot plate and brought to a boil with agitation of the slurry. The slurry is boiled for five minutes and then placed in 90 °C water and incubated for two hours. After the boil, an additional 1.2 ml of the enzyme Taka-Therm.RTM. Il is added to the slurry. The slurry is cooled to 25 °C and the pH adjusted to 4.6-4.8 with 25% H₂SO₄. The dry solid level (DS) is adjusted to 20-21 % with tap water.
   The saccharification and fermentation are carried out simultaneously in 500 ml Erlenmeyer flasks by adding 450 gm of the liquefied corn mash obtained in step a (liquefaction). The appropriate amount of enzymes, 0.25 ml distillase L-200 and 0.3 ml 2% solution of acid fungal protease, are then added to the mash along with 0.8 gm of Fleishmann's bakers yeast (7 gm foil package). The dry yeast is allowed to hydrate by about 10 minutes prior to swirling the flasks to mix in the yeast. The flasks are then covered with Parafilm and placed in a 36 °C water to allow fermentation for 24 hours. The ethanol was then recovered.
b. Example 4a was repeated using corn meal produced from corn derived from a waxy maize plant which is heterozygous for the recessive sugary-2 allele (one dose).
c. Example 4a was repeated using corn meal produced from corn derived from a waxy maize plant which is heterozygous for the recessive sugary-2 allele (two doses).
Ethanol production is similar to that by yeast grown on waxy and dent corn grain samples with less energy input. Example 4a required the lowest energy input.

### Example 5 - Citric acid production

a. Solvent extracted corn meal produced from corn derived from a waxy maize plant which is homozygous for the recessive sugary-2 allele is a rich source of starch for fermentation. One method to provide soluble sugars suitable for fermentation is to hydrolyze starch molecules. Types of enzymes that can be useful to convert the starch and protein matrix of corn meal into simple sugars suitable for fermentation include amylase(s), proteases, cellulase(s) (e.g., xylonase), esterase(s) (e.g., ferulase, acetylesterase) and ligninase(s). The corn meal is ground to pass through a 1 mm screen using a Retsch Mill and 300 g was combined with 700 ml of water at 100 °C containing 0.5 ml alpha-amylase and placed in a sealed container. The pH was adjusted to 5.9 with base and stirred for 45 min and additional .alpha.-amylase enzyme was added. After an additional 45 min of incubation, the pH is adjusted to 4.5 with acid. One-half of one milliliter (0.5 ml) glucoamylase (Optimax 7525) and 0.5 g protease (Fungal Protease 5000) are added and incubated with both enzymes at 62 °C for about 24 hours. Throughout the procedure, the degree of starch hydrolysis is monitored by HPLC (Waters 2690 Separations module) using an organic acid column (Aminex HPX-87H lon Exclusion Column, 300 mm.times.7.8 mm, Bio Rad).
   Once the starch is suitably prepared through treatment with enzymes, the solution is filtered and demineralized according to commonly known practices. Resulting sugars are brought to a solids content of about 120 mg/l with demineralized water in a deep-tank fermentation vessel. The deep tank method is also known as the submerged process. In this method the tank is supplied with sterile air, nutrients and a carbon source, (hydrolyzed starch), and inoculated with Aspergillus niger spores. Spores of the fungus in a concentration of about 100 spores per liter of culture liquid, which corresponds to an amount of 10 to 15 g of spores per cubic meter (m.sup.3) would be added to the nutrient solution and the citric acid production would be carried out by the fungus. Examples of A. niger strains are ATCC 1015 described in U.S. Pat. No. 2,492,667, and DSM 5484 described in U.S. Pat. No. 5,081,025.
   The incubation of the broth thus inoculated would be carried out at conditions generally known and described for citric acid production, such as continued aeration and temperature control. During the fermentation process, the temperature would be maintained at about 32 °C, the pH would be maintained at about 2 to 3 with sodium citrate, and sterile air would be added to maintain about 50% dissolved oxygen content. Fermentation would be carried out until the fermentation broth reaches a reducing sugar content of about 1 g/L, which may require several days to achieve. Two main separation processes can be used in the recovery of citric acid, the Lime-Sulfuric Acid process and the Liquid extraction process. The Lime-Sulfuric Acid method is commonly used and is familiar to those skilled in the art of citric acid production.
b. Example 5a was repeated using corn meal produced from corn derived from a waxy maize plant which is heterozygous for the recessive sugary-2 allele (one dose).
c. Example 5a was repeated using corn meal produced from corn derived from a waxy maize plant which is heterozygous for the recessive sugary-2 allele (two doses).

## Claims

1. In a method of producing fermentation-based products from corn of the type wherein the corn is mixed with a micro-organism capable of fermenting a carbon source to produce fermentation-based products, the improvement comprising using a corn comprising a starch **characterized by** a gelatinization temperature of less than about 60 °C.

2. The method of claim 1, wherein the corn is from a maize plant which endosperm is homozygous recessive for the allele selected from the group consisting of sugary2, sugary2-waxy, dull-sugary2, dull-sugary2-waxy, amylose extender-dull-waxy, amylose extender-sugary2, and dosage combinations thereof

3. The method of claim 1 or 2, wherein the corn is from a waxy maize plant which endosperm is homozygous for the recessive sugary-2 allele.

4. The method of claim 1 or 2, wherein the corn is from a waxy maize plant which endosperm is heterozygous for the recessive sugary-2 allele, one dose.

5. The method of claim 1 or 2, wherein the corn is from a waxy maize plant which endosperm is heterozygous for the recessive sugary-2 allele, two doses.

6. The method of any one of claims 1-5, wherein the starch is **characterized by** a gelatinization temperature of less than about 55 °C.

7. The method of claim 6, wherein the starch is **characterized by** a gelatinization temperature of less than about 50 °C.

8. The method of any one of claims 1-7, wherein the fermentation-based product is selected from the group consisting of ethanol, citric acid, butanol, hydrogen, lactic acid, vitamins, and soluble sugars.

9. The method of claim 8, wherein the fermentation-based product is ethanol.

10. The method of claim 8, wherein the fermentation based product is citric acid.
